# EUROPÄISCHE PATENTSCHRIFT

(11) **EP 0 897 718 B1**
(45) Veröffentlichungstag und Bekanntmachung des Hinweises auf die Patenterteilung: **11.04.2001**
(21) Anmeldenummer: 98115114.5
(22) Anmeldetag: 11.08.1998
(51) Int. Cl.: A61K 7/50, A61K 7/48

(54) **Verwendung von abspülbaren Öl-in-Wasser Emulsionen zur Entfernung und Vermeidung von Comedonen und dadurch zur Akneprophylaxe**
Use of rinsable oil-in-water emulsions for removal and prevention of comedones and thereby for acne prophylaxis
Utilisation des émulsions huile-dans-eau rinçables pour l'élimination et la prévention des comédones pour la prophylaxie de l'acné.

(30) Priorität: 16.08.1997 DE 19735518
(43) Veröffentlichungstag der Anmeldung: 24.02.1999
(73) Patentinhaber: Geier, Friedrun, 66740 Saarlouis (DE)
(72) Erfinder: Geier, Friedrun, 66740 Saarlouis (DE)

(56) Entgegenhaltungen:
- EP-A- 0 117 080
- EP-A- 0 806 201
- WO-A-98/51275
- WO-A-98/52528
- DE-A- 19 719 856
- US-A- 4 857 321
- US-A- 5 618 522
- US-A- 5 925 348
- BREITMAIER ET AL: "Organische Chemie II", 1983, G.THIEME VERLAG, STUTTGART

## Beschreibung

Die vorliegende Erfindung betrifft die Verwendung von talglösenden topischen Zubereitungen zur Entfernung von Comedonen, zur Vermeidung von Comedonbildung sowie zur Prophylaxe von Akne.

Talgdrüsenüberproduktion und die möglichen sich daraus entwickelnden Hautstörungen wie Talgretention, Ausbildung von Comedonen im Gebiet der Talgdrüsen, also im Gesicht (vor allem Stirn, Nase und Kinn) und auf dem oberen Rücken und in der Folge die verschiedenen Formen der Akne, sind ein häufig anzutreffendes Hautproblem, das bisher nicht in zufriedenstellendem Maße gelöst worden ist.

Talg ist das Sekret der Talgdrüse. Talgdrüsen sind Ausbuchtungen des Follikelepithels und damit Bestandteile des Follikels, mit dem sie eine funktionelle Einheit bilden. Sie sind holokrine Drüsen, das bedeutet, die ganze Drüsenzelle wird zum größten Teil in das Sekret Talg umgewandelt. Die Zellen der Talgdrüse, die von unten her ständig erneuert werden, verfetten und zerfallen, und der so gebildete Hauttalg wird durch die Follikelmündungen auf die Hautoberfläche entleert.

Der Hauttalg besteht nach G. Leonardy (J.Ft. Jellinek Kosmetologie, Zweck und Aufbau kosmetischer Präparate, Dr. Alfred Hüthig-Verlag, Heidelberg - Mainz - Basel, dritter völlig überarbeiteter und wesentlich erweiterte Auflage 1976, Seiten 26 bis 29) aus Mono-, Di- und Triglyceriden (C₁₀-C₁₈), Wachsen (C₁₆-C₂₆), Wachsestern (C₂₈-C₃₈), normalen gesättigten Fettsäuren (C₁₀-C₁₈), verzweigtkettigen gesättigten Fettsäuren (C₁₁-C₁₈), mehrfach verzweigtkettigen gesättigten Fettsäuren (C₁₃-C₁₈), einfach ungesättigten Fettsäuren (C₁₁-C₁₈), mehrfach ungesättigten Fettsäuren (C₁₃,
C₁₅-C₁₇), Sterolen (Cholesterin, 7-Dehydrocholesterin, 7-Hydroxycholesterin), verzweigten und unverzweigten Kohlenwasserstoffen (C₃₀-C₄₀), Squalen und Phospholipiden.

Zusammen mit dem wäßrigen Sekret der ekkrinen Schweißdrüsen bilden die Lipide der Talgdrüse den sogenannten Hydro-Lipid-Film der Haut. Dieser Oberflächenfilm ist eine Emulsion, die eine Wasser-in-Öl oder eine Öl-in-Wasser-Emulsion sein kann. Sie hat die Funktion, die Hautoberfläche geschmeidig zu halten und den Wassergehalt der tieferliegenden Hautschichten zu regeln. Bei guter Hydratation des Sebums beträgt der Wassergehalt mindestens 10 bis 20 Gew.-% und das Sebum ist hydrophil. Ist das hydrophillipophile Gleichgewicht des Oberflächenfilmes nicht mehr gegeben und der Wassergehalt sinkt, so verändert sich das Sebum und wird hydrophob. Der Talgabfluß aus Talgdrüse und Follikel wird behindert. Es kommt zu einer Stauung des Hauttalgs in den Follikelmündungen, die dann in der Folge zu Comedonen und Entzündungen der Follikel führen kann.

Die Veränderung des Hauttalgs und das Einleiten der Comedonenbildung kann verschiedene Ursachen haben. Zum Beispiel: äußere Einflüsse, wie falsche Reinigungsgewohnheiten und falsche Pflege, comedogene Substanzen in Kosmetika, Witterungseinflüsse, alkalische Seifen, scharfe Detergentien. Eine vermehrte Talgdrüsensekretion und die Ausbildung von Comedonen kann sich auch durch genetische Faktoren und hormonelle Einflüsse entwickeln. Auch hier können Talgretensionen, Comedone, Entzündungen, Prä-Akne und Akne mit ihren Nachwirkungen die Folge sein.

Die Häufigkeit der Hautschäden durch eine gestörte Talgdrüsenfunktion und Krankheiten der Talgdrüsen steigen immer mehr, und die Rückführung/Vermeidung der Comedonenbildung ist somit ein vordringliches Anliegen. Doch bisherige Versuche, die Comedonenbildung als ein ursächliches Problem zu lösen, haben zu wenig befriedigenden Ergebnissen geführt.

Neben dem manuellen Entfernen der Comedone durch Ausdrücken sind zahlreiche Reinigungsmethoden bekannt, mit denen versucht wird, Comedone zu entfernen und die Comedonenbildung nachhaltig zu verhindern. Hierzu gehören spezielle Seifen, hautabschälende Kompositionen und dergleichen. Erweichende und adstringierende Mittel finden ebenso Anwendung.

Darüber hinaus wird versucht, durch Zusatz von austrocknenden, keratolytischen, antiseborrhoeschen und antibakteriellen Wirkstoffen in kosmetischen und pharmazeutischen Zubereitungen die Neigung zur Akne zu reduzieren, ohne daß sich Irritationen der Haut oder ein Austrocknen der Haut einstellen.

US-A-4857321 beschreibt Zusammensetzungen, welche Tenside und tierische und pflanzliche Öle enthalten. U.a. wird die Akne-Behandlung genannt. Die Beschreibung und Vermeidung von Comedonen wird nicht erwähnt.

Die Hautreinigung entfettet allerdings die Haut und entzieht ihr Feuchtigkeit. Zusätzlich haben Seifen den Nachteil, daß die sich bei Verwendung der Seifen in hartem Wasser bildenden wasserunlöslichen Calcium- und Magnesiumsalze der höheren Fettsäuren auf der Haut schleimige Niederschläge bilden. Diese Niederschläge verbleiben bei schlechter Abspülbarkeit länger auf der Haut, verstopfen die Follikelmündungen und können zu einer Ausbildung von Comedonen führen. Daher werden zur Hautreinigung vorwiegend Syndets (d.h. Tenside ohne Seifencharakter) in Form von Waschcremes oder Waschlotionen eingesetzt. Diese Syndets bilden zwar keine Kalkseifen, aber die Behandlung mit stark oberflächenaktiv wirkenden Agentien hat auf die Haut eine stärker entfettende und austrocknende Wirkung als Seife. Je häufiger seifen- und tensidhaltige Produkte auf der Haut angewandt werden, desto deutlicher treten deren nachteilige Wirkungen, nämlich Entfettung und Austrocknung der Haut durch Zerstörung des Hydro-Lipid-Filmes in den Vordergrund. Fast immer führt die Reduzierung der Comedone zur Absenkung des Wassergehaltes in den oberen Hautschichten und zu einer festen Konkrementbildung in den Talgdrüsen, die wiederum Entzündungen induzieren kann. Die Absenkung des Feuchtigkeitsgehaltes der Haut ist aber für eine pflegende Entfernung der Comedone kontaproduktiv.

Der vorliegenden Erfindung liegt die Aufgabe zugrunde, eine Zubereitung zur Verfügung zu stellen, die die Nachteile der bekannten und bisher verwendeten Mittel nicht aufweist, die gleichzeitig talglösend, fettregulierend und pflegend wirkt, die bereits vorhandenen Comedone ohne Irritationen entfernt, die Entfettung und Austrocknung der Haut wirksam verhindert, die Comedonenbildung und die Entstehung von Akne Kosmetika verhindert, sowie schon bestehende Akne bessert und die gleichzeitig die Haut pflegt.

Diese Aufgabe wird erfindungsgemäß durch eine Verwendung mit den kennzeichnenden Merkmalen gemäß Patentanspruch 1 gelöst. Überraschenderweise wird bei Verwendung der Zubereitung die comedogene Wirkung der in der Zubereitung verwendeten Rohstoffe aufgehoben und damit die Bildung von Comedonen und entsprechend die Entstehung von Akne Kosmetika verhindert.

Die Zubereitung stellt eine Emulsion dar, die in Form einer Öl-in-Wasser-Emulsion oder in Form einer amphiphilen Emulsion vorliegt. Die Konsistenz der Zubereitung kann fest, flüssig oder cremeartig sein. Für den kosmetischen und/oder pharmazeutischen Bereich bietet es sich an, für die Zubereitung eine cremeartige Konsistenz vorzusehen. Eine derartige Ausführungsform hat das beste Eindringungsvermögen in die Haut und läßt sich dann bei der Anwendung besonders leicht handhaben.

Die Zubereitung enthält mindestens eine waschaktive Substanz. Vorzugsweise sind jedoch mehrere waschaktive Substanzen in den Zubereitungen enthalten. Als waschaktive Substanzen geeignet sind anionische und/oder nicht-ionische und/oder amphotere Substanzen.

Beispiele für anionische Substanzen sind:
Salze von höheren Fettsäuren, wie Natrium-, Kalium- und Aminsalze, wie Triethanolaminsalze, d.h. Seifen, besonders der C₁₂-C₁₈-Fettsäuren (wie Laurinsäure, Myristinsäure, Ölsäure, Palmitinsäure, Stearinsäure), besonders bevorzugt sind C₁₆-C₁₈-Fettsäureseifen,
sulfierte Produkte, wie Fettalkoholethersulfate (Alkylethersulfate), wie Natriumlaurylethersulfat, Natriumlauryl-diglykolethersulfat, Natriummyristylethersulfat,
Fettsäure-Polypeptid-Kondensationsprodukte, wie das Kondensationsprodukt von Eiweißhydrolysat aus natürlichem Collagen mit Kokosfettsäuren;

Beispiele für nicht-ionische Substanzen sind:
Glycerinfettsäureester-ethoxylate, wie höher ethoxylierte Mono-/di-glyceride der Talgfettsäure,
Saccharosefettsäureeste, wie Saccharoseester mit Fettsäureestern auf der Basis von Kokosfettsäure;

Beispiele für amphotere Substanzen sind:
Betaine.

Besonders geeignet sind Salze von höheren Fettsäuren und insbesondere geeignet sind die Kaliumsalze (Kaliumseifen) - allein oder im Gemisch mit anderen waschaktiven Substanzen, vorzugsweise im Gemisch mit anderen waschaktiven Substanzen.

Bezogen auf die Gesamtzusammensetzung der Zubereitung kann die Menge an waschaktiver Substanz in der Zubereitung 5 bis 60 Gew.-%, vorzugsweise 10 bis 30 Gew.-%, betragen. Bei einem Gehalt unter 5 Gew.-% waschaktiver Substanz, bezogen auf die Gesamtzusammensetzung der Zubereitung, ist eine gute talglösende Wirkung nicht mehr gewährleistet. Bei einem Gehalt über 60 Gew.-% waschaktiver Substanz ist das Eindringungsvermögen und die pflegende Wirkung nicht mehr gegeben. Desgleichen kann die Wirkung der Zubereitung durch einen höheren Gehalt an waschaktiver Substanz nicht gesteigert werden.

Unter dem in der Zubereitung verwendeten Wasser wird reines Wasser verstanden. Des weiteren kann das reine Wasser auch ganz oder teilweise durch beispielsweise Rosenwasser, Hamameliswasser oder Krauseminzwasser ersetzt werden. Der Wassergehalt beträgt in Abhängigkeit von der gewünschten Ausführungsform der Zubereitung mindestens 25 Gew.-% und kann vorzugsweise zwischen 25 und 75 Gew.-% variieren.

Als Lipid kann in der Zubereitung mindestens ein Lipid, vorzugsweise ein Gemisch aus zwei oder mehreren verschiedenen Lipiden verwendet werden, wobei jedes pflanzliche oder tierische Lipid sowie dessen synthetische Ersatzprodukte oder Derivate eingesetzt werden können, sofern sie in ihren Eigenschaften dem natürlichen Lipid gleichwertig sind.

Beispiele für Lipide sind:
pflanzliche Öle und/oder Fette, wie Olivenöl, Avocadoöl, Weizenkeimöl, Pfirsichkernöl, Ringelblumenöl, Erdnußöl, Sesamöl, Sojaöl, Mandelöl, Sheabutter, Kakaobutter, Unverseifbares der Avocado, vorzugsweise Olivenöl, Avocadoöl, Mandelöl oder Sheabutter,
tierische Öle und/oder Fette, wie vorzugsweise Nerzöl oder Walratöl,
pflanzliche und/oder tierische Wachse, wie Jojobaöl, Bienenwachs, Perhydrosqualen, Purcellinöl, Walratersatz, vorzugsweise Jojobaöl, Bienenwachs, Purcellinöl oder Walratersatz,
Fettalkohole, wie vorzugsweise Cetylalkohol,
Sterole, wie Phytosterine, und/oder
Phospholipide, wie Lecithin,
die einzeln oder in Form von Gemischen aus zwei oder mehreren der vorstehend genannten Lipiden eingesetzt werden können. Insbesondere geeignet ist jedoch ein Gemisch aus zwei oder mehreren der genannten verschiedenen Lipide.

Eine besonders bevorzugte Lipidkombination sollte eine dem natürlichen Hauttalg ähnliche Zusammensetzung aufweisen. Derartige Ausführungsformen der Zubereitung besitzen eine besonders gute eindringende, weichmachende, pflegende und glättende Wirkung auf die Haut.

Der Gehalt an Lipiden kann, bezogen auf die Gesamtzusammensetzung der (anwendungsfertigen) Zubereitung, 5 bis 60 Gew.-%, vorzugsweise 10 bis 30 Gew.-%. betragen. Bei einem Gehalt von unter 5 Gew.-% Lipid ist eine pflegende Wirkung nicht ausreichend gegeben, während bei einem Gehalt über 60 Gew.-% an Lipiden das Eindringungsvermögen in die Haut reduziert ist und die pflegende Wirkung nicht verbessert werden kann.

Bei der Zubereitung kann durch eine unterschiedliche Kombination und Konzentration der Lipide die pflegende Wirkung der Zubereitung variiert werden. Des weiteren kann durch eine unterschiedliche Kombination und Konzentration der waschaktiven Substanzen die fettregulierende Wirkung der Zubereitung variiert werden.

Gemäß einer weiteren Ausführungsform können die Zubereitungen einen oder mehrere unterschiedliche Wirkstoffe enthalten. Als Wirkstoffe können grundsätzlich alle hydrophilen und lipophilen Substanzen verwendet werden, die von der Zubereitung eingelagert werden können. Besonders geeignet sind Wirkstoffe, die keratolytisch, antibakteriell oder weichmachend wirken, wie beispielsweise Harnstoff, Allantoin, Natriumlactat, Salicylsäure und/oder schwefelhaltige Verbindungen. Derartige Ausführungsformen eignen sich besonders zur Behandlung seborrhoescher Haut und den unterschiedlichen Formen der Akne.

Die Zubereitungen können entsprechend der gewünschten Ausführungsform Hilfs- und/oder Zusatzstoffe enthalten. Beispielsweise können Substanzen verwendet werden, welche die Emulsion durch Verdicken der Wasserphase stabilisieren, wie zum Beispiel Methylcellulose, Alginate, wie Natriumalginat, Carragenate, oder dispergierte Feststoffe, die als inerte Füllmittel, Dispergiermittel oder Hilfsemulgatoren die Konsistenz der Zubereitung festigen und die Emulsion stabilisieren, wie zum Beispiel Bentonit, Stärke, Talk, sowie Mittel zur pH-Einstellung, zum Beispiel α-Hydroxysäuren, wie Milchsäure, Fruchtsäuren, wie Äpfelsäure, Weinsäure, Zitronensäure, oder lecithinähnliche Phosphorsäureester. Der Gehalt an Hilfs- und Zusatzstoffen kann, bezogen auf die Gesamtzusammensetzung der Zubereitung 0,1 Gew.-% bis 10 Gew.%, vorzugsweise 0,5 bis 5 Gew.%, betragen.

Der Gehalt an Wirkstoffen, Hilfs- und Zusatzstoffen kann, bezogen auf die Gesamtzusammensetzung der Zubereitung, 0,1 bis 30 Gew.-%, vorzugsweise 0,5 bis 15 Gew.-%, betragen.

Ätherische Öle können in den Zubereitungen als Wirkstoffe mit fungizider und/oder bakterizider Wirkung eingesetzt werden. Sie können zur Unterstützung der Konservierung dienen und diese zum Teil ersetzen. Beispiele für geeignete ätherische Öle sind Eukalyptusöl, Zitronenöl, Salbeiöl, Minzeöl und Benzoeharz. Ätherische Öle können auch zur Parfümierung in den Zubereitungen eingesetzt werden. Beispiele für solche Öle sind Zitronenöl, Eukalyptusöl, Lavendelöl, Minzeöl, Rosenöl.

Gemäß einer weiteren Ausführungsform können die Zubereitungen ein oder mehrere natürliche oder synthetische Schleifmittel, vorzugsweise ein Gemisch verschiedener natürlicher und/oder synthetischer Schleifmittel enthalten. Zubereitungen gemäß dieser Ausführungsform eignen sich zur physikalischen (mechanischen) Entfernung der obersten Hornschichtlagen. Indiziert sind solche Ausführungsformen besonders bei Hyperkeratosen und Prä-Akne. Als abrasive Substanzen können zum Beispiel Polyethylengranulat, Nußschalengranulate, wie pulverisierte Walnußschalen, Aprikosenkernmehl und/oder Jojobagranulat, vorzugsweise Polyethylengranulat und/oder Nußschalengranulate, enthalten sein. Der Gehalt an Schleifinittel kann, bezogen auf die Gesamtzusammensetzung der Zubereitung, 1 bis 30 Gew.-%, vorzugsweise 5 bis 15 Gew.%, betragen.

Entsprechend den Anteilen der in den Zubereitungen enthaltenen Lipide sind in den Zubereitungen Fettstabilisatoren als Oxidationsschutz, vorzugsweise Antioxidantien mit Synergisten, wie beispielsweise α-Tocopherol mit Zitronensäure, Weinsäure und/oder ihren Salzen, enthalten.

Weiterhin können die Zubereitungen auch Substanzen enthalten, die sie vor der Einwirkung von Bakterien und Schimmelpilzen schützen, wie zum Beispiel Ester der p-Hydroxybenzoesäure oder Adamantan-Derivate.

Durch den Gehalt der Zubereitungen an unterschiedlichen Wirkstoffen, Hilfs- und Zusatzstoffen können die kosmetischen und/oder pharmazeutischen Anwendungen der Zubereitungen vervielfältigt werden.

Wie bereits vorstehend genannt, kann die (kosmetische und/oder pharmazeutische) Zubereitung in Abhängigkeit der gewünschten Anwendungsform flüssig, cremeartig oder fest sein. Entscheidend hierfür ist die zur Herstellung der Zubereitung verwendete Kombination grenzflächenaktiver Substanzen und Lipide sowie deren Gehalt in den Zubereitungen. Besonders geeignet ist eine Zubereitung von cremeartiger Konsistenz, da eine solche Ausführungsform ein hohes Eindringungsvermögen in die Haut besitzt. Dies wiederum führt dazu, daß sowohl eine gute fettregulierende als auch eine pflegende Wirkung gewährleistet ist.

Um die erforderliche Abspülbarkeit der Zubereitung zu gewährleisten, soll die Emulsionsform eine Öl-in-Wasser-Emulsion oder eine amphiphile Emulsion sein. Die in der Zubereitung verwendeten grenzflächenaktiven Substanzen und Lipide wirken als anionische und/oder amphotere und/oder nicht-ionische Emulgatoren. Ebenso wirken die entsprechend der gewünschten Anwendungsform eingesetzten quasi Emulgatoren (Hilfsemulgatoren) und/oder Stabilisatoren, wie beispielsweise Methylcellulose, Alginate, Bentonite.

Darüber hinaus können zusätzlich auch andere Emulgatoren in der Zubereitung enthalten sein. Insbesondere geeignet sind beispielsweise Mono-/di-fettsäureester mehrwertiger Alkohole oder deren Derivate, wie Glycerinmono/di-stearat, Lanettecremes, Natrium- oder Triethanolaminsalz eines Fettalkoholsulfonats, vorzugsweise Monofettsäureester mehrwertiger Alkohole oder Glycerinmono/di-stearat. Der Gehalt dieser Emulgatoren in der Zubereitung liegt zwischen 0,5 und 10 Gew.-%, und beträgt, vorzugsweise 1 bis 5 Gew.-%. Solche Emulgatoren gewährleisten eine Öl-in-Wasser-Emulsion oder eine amphiphile Emulsion. Dies wiederum führt dazu, daß bei der Anwendung die gewünschte Abspülbarkeit der Zubereitung erhalten bleibt.

Die Zubereitungen weisen eine Reihe von Vorteilen auf. So werden bei Verwendung der Zubereitung im Vergleich zu den herkömmlichen, zur Behandlung von Comedonen, erhöhter Talgdrüsensekretion und verschiedenen Formen der Akne eingesetzten Zubereitungen durch die Hautpflege auf die Haut gebrachten comedogenen Substanzen leicht entfernt. Darüber hinaus werden durch die Zubereitung die Calcium- und Magnesiumniederschläge der höheren Fettsäuren, die durch die Verwendung von Seife in hartem Wasser auf der Haut einen Film bilden, aufgelöst.

Die Anwendung der hergestellten Zubereitung wirkt fettregulierend bei erhöhter Talgdrüsensekretion und talglösend auf bestehende Comedone. Dies wiederum führt dazu, daß bei Anwendung einer Zubereitung die Entstehung einer durch Kosmetika verursachten Akne wirksam verhindert sowie schon bestehende Akne verbessert wird.

Des weiteren konnte festgestellt werden, daß der durch die Behandlung mit stark oberflächenaktiv wirkenden Agentien reduzierte Feuchtigkeitsgehalt der Haut und die dadurch bedingte Zerstörung des Hydro-Lipid-Filmes durch Austrocknung und Entfettung der oberen Hautschichten und des Hauttalges gebessert und ausgeglichen werden. Durch die sehr weichmachende Wirkung der Zubereitung werden Talgretentionen gelockert und ausgeleitet und die Haut wird bleibend verbessert. Gleichzeitig wird die Haut weich, geschmeidig und glatt.

Darüber hinaus weist die Zubereitung eine hohe Hautverträglichkeit auf und ermöglicht somit eine schonende Anwendung auf die Haut, so daß Irritationen, Austrocknung und Entfettung der Haut selbst bei solchen Substanzen nicht auftreten, die als Reinsubstanzen bei einer topischen Anwendung erhebliche Hautirritationen hervorrufen. Diese hohe Hautverträglichkeit ermöglicht die Anwendung der Zubereitung selbst bei Atopikern.

Die Zubereitungen können wirksam zur Prophylaxe von vorzugsweise Akne, eingesetzt werden. Desgleichen finden sie wirksam Verwendung zur Entfernung von Comedonen und zur Vermeidung einer Comedonenbildung sowie zur Entfernung der oberen Hornschichtlagen der Haut bei Hyperkeratosen und Prä-Akne.

Die Herstellung der Zubereitungen erfolgt in an sich bekannter Weise.

Die in der vorliegenden Beschreibung aufgeführten Konzentrationsangaben (Gewichtsprozent) beziehen sich auf die Gesamtzusammensetzung der Zubereitung, es sei denn, ein anderer Bezug ist ausdrücklich offenbart.

Die nachfolgenden Beispiele dienen der Erläuterung der vorliegenden Erfindung.

### Beispiel 1

Aus den nachfolgend angegebenen Bestandteilen wurde eine Zubereitung hergestellt.

| **Bestandteile** | | **ca. Gew.-%** | **Funktion** |
|---|---|---|---|
| A. | Kaliumseife auf der Basis von Baumwollsamenöl stabilisiert durch Kartoffelstärke Fettsäure ca. 40 % | 35.0 | ca. 24 Gew.-% anionische, amphotere und nichtionische waschaktive Substanzen |
| | Natriumlaurylethersulfat + 5 Gew.-% Betain 56 %ig | 5.0 | |
| | Sacccharoseester mit Kokosfettsäureester 70 %ig | 5.0 | |
| | Höheretoxylierte Mono/di-glyceride der Talgfettsäure 70 %ig | 5.0 | |
| B. | Olivenöl | 1.5 | 11.5 Gew.-% einer Lipidmischung aus pflanzlichen Ölen und Fetten, Phytosterine, Phospholipide tierischen Wachsen Wachsersatz, Fettalkobole |
| | Sojaöl | 2.0 | |
| | Avocadoöl | 1.0 | |
| | Sheabutter | 0.5 | |
| | Unverseifbares der Avocado | 0.5 | |
| | Lecithin | 2.0 | |
| | Bienenwachs | 1.5 | |
| | Walratersatz | 1.0 | |
| | Cetylalkohol | 1.5 | |
| | α-Tocopherol mit Weinsäure | 2.0 | Fettstabilisator |
| | Molekulardestilliertes Glycerinmonostearat selbstemulgierend | 1.5 | Emulgator |
| C. | Harnstoff | 2.0 | Wirkstoff |
| | Wasser demin. u. konserv. | 10.0 | |
| D. | Natriumalginat | 0.5 | Konsistenzgeber |
| | Wasser demin. u. konserv. | 10.0 | |
| E. | CMC. Carboxymethylcellulose 3000mPas | 0.3 | Konsistenzgeber |
| | Wasser entminer. u. konserv. | 10.0 | |
| F. | Lavendelöl | 0.5 | Wirkst./Parfümierung zur pH-Einstellung |
| G. | Weinsäure | | |

Die Herstellung der Zubereitung erfolgte in folgender Weise:

Die unter A und die unter B genannten Substanzen wurden separat gemischt und auf 70°C erwärmt.
Die unter C, D und E genannten Substanzen wurden separat zusammengemischt und unter weiterem Erwärmen nach und nach in das durch Vermischen der unter A genannten Substanzen erhaltene Gemisch eingerührt.
Das so erhaltene Gemisch wurde langsam unter Rühren dem etwa 70°C warmen Gemisch der Bestandteile B zugesetzt. Es wurde weitergerührt bis das so erhaltene Gemisch auf etwa 40°C abgekühlt war. Anschließend wurde der unter F genannte Bestandteil zugesetzt und danach mit dem unter G genannten Bestandteil der pH-Wert eingestellt.

### Beispiel 2

Aus den nachfolgend angegebenen Bestandteilen wurde eine Zubereitung hergestellt.

| **Bestandteile** | | **ca. Gew.-%** | **Funktion** |
|---|---|---|---|
| A. | Kaliumseife auf der Basis von Baumwollsamenöl stabilisiert mit Kartoffelstärke Fettsäure ca. 40 % | 25.0 | 17 Gew.-% einer Kombination waschaktiver Substanzen |
| | Natriumlaurylethersulfat + 5 Gew.-% Betain 56 %ig | 6.0 | |
| | Höheretoxylierte Mono/di-glyceride der Talgfettsäure 70 %ig | 5.0 | |
| B. | Wasser entmin. konserv. | ca. 25.0 | |
| C. | Bienenwachs | 1.5 | 17.5 Gew.-% einer Lipidmischung aus tierischen Wachsen, Wachsersatz, pflanzlichen Ölen, Fettalkohol |
| | Walratersatz | 1.5 | |
| | Olivenöl | 2.5 | |
| | Süßmandelöl | 6.0 | |
| | Ringelblumenöl | 1.5 | |
| | Cetylalkohol | 4.5 | |
| | α-Tocopherol mit Zitronensäure | 2.5 | Fettstabilisator |
| | Glycerinmono/di-stearat und Zitronensäureester von Mono/di-glyceriden | 3.0 | Emulgator |
| D. | Allantoin | 0.5 | Wirkstoff |
| | Wasser entminer. konserv. | 5.0 | |
| E. | Mandelsteingranulat | 3.0 | Schleifmittel |
| | Taguanußgranulat | 1.0 | |
| | Jojobagranulat | 2.0 | |
| F. | Lavendelöl | 0.5 | Wirkst./Parfümierung |
| G. | Zitronensäure | | zur pH-Einstellung |

Die Herstellung dieser Zubereitung wurde in folgender Weise durchgeführt:

Die unter A genannten Bestandteile wurden unter Rühren auf etwa 70°C erwärmt. Anschließend wurde der Bestandteil B in die so erwärmten Bestandteile A unter weiterem Erwärmen bis auf etwa 70°C eingerührt. Die Bestandteile C wurden auf etwa 70°C erwärmt. Anschließend wurde das Gemisch aus den Bestandteilen A und B langsam unter Rühren dem 70°C warmen Gemisch der Bestandteile C zugesetzt. Das so erhaltene Gemisch wurde anschließend unter Rühren auf etwa 40°C abgekühlt und danach mit den vermischten Bestandteilen D versetzt. In das so erhaltene Gemisch wurden die Bestandteile E eingerührt, dann der Bestandteil F zugesetzt und danach der pH-Wert des so erhaltenen Gemisches mit dem Bestandteil G eingestellt.

### Beispiel 3

Aus den nachfolgenden Bestandteilen wurde eine Zubereitung hergestellt.

| **Bestandteile** | | **ca. Gew.-%** | **Funktion** |
|---|---|---|---|
| A. | Natriumseife auf der Basis von Olivenöl Fettsäure ca. 70 % | 20.0 | ca. 23 Gew.-%, waschaktive Substanzen |
| | Natriumlaurylethersulfat + 5 Gew.-% Betain 56 %ig | 10.0 | |
| | Höheretoxylierte Mono/di-glyceride der Talgfettsäure 70 %ig | 5.0 | |
| B. | Harnstoff | 2.0 | Wirkstoff |
| | Wasser entmin. u. konserv. | 30.0 | |
| C. | CMC. Carboxymethylcellulose | 0.3 | Konsistenzgeber |
| | Wasser entmin. u. konserv. | 20.0 | |
| D. | Olivenöl | 1.0 | 10 Gew.-% einer Lipidkombination aus pflanzlichen Ölen und Fetten, Fettalkohol, Phospholipid |
| | Mandelöl | 1.5 | |
| | Sesamöl | 1.5 | |
| | Sojaöl | 2.0 | |
| | Kakaobutter | 0.5 | |
| | Lecithin | 2.0 | |
| | Cetylalkohol | 1.5 | |
| | α-Tocopherol mit Weinsäure | 1.0 | Fettstabilisator |
| | Molekulardestilliertes Glycerinmonostearat selbstemulgierend | 1.0 | Emulgator |
| E. | Zitronenöl, Krauseminzöl | 0.5 | Wirk./Parfümierung zur pH-Einstellung |
| F. | Milchsäure | | |

Die Herstellung der Zubereitung erfolgte in folgender Weise:

Die Bestandteile A wurden auf etwa 70°C erwärmt und vermischt. Separat wurden jeweils die Bestandteile B und C gemischt und anschließend die so separat gemischten Bestandteile B und C unter weiterem Erwärmen und Rühren dem Gemisch der Bestandteile A zugesetzt. Die Bestandteile D wurden auf etwa 70°C erwärmt und anschließend nach und nach unter Rühren mit dem etwa 70°C warmen Gemisch der Bestandteile A, B und C versetzt. Das so erhaltene Gemisch wurde bis zur Abkühlung auf etwa 40°C weitergerührt. Anschließend wurden die Bestandteile E zugesetzt und der pH-Wert des so erhaltenen Gemisches mit dem Bestandteil F eingestellt.

### Beispiel 4

Aus den nachfolgend angegebenen Bestandteilen wurde eine Zubereitung hergestellt.

| **Bestandteile** | | **ca. Gew.-%** | **Funktion** |
|---|---|---|---|
| A. | Kaliumseife auf der Basis von Leinöl, Fettsäure ca. 40 % | 30.0 | ca. 17 Gew.-% waschaktive Substanzen |
| | Kondensationsprodukt von Eiweiß-hydrolysat aus natürlichem Kollagen mit Kokosfettsäuren 39 %ig | 5.0 | |
| | Natriumlaurylethersulfat + 5 Gew.-% Betain 70 %ig | 5.0 | |
| B. | Harnstoff | 2.0 | Wirkstoff |
| | Wasser entmin. u. konserv. | 10.0 | |
| C. | Kolloidaler Schwefel | 2.0 | Wirkstoff |
| | Wasser entmin. u. konserv. | 10.0 | |
| D. | Bentonit | 0.2 | Konsistenzgeber |
| | Wasser entmin. u. konserv. | 10.0 | |
| E. | Methylhydroxycellulose DAB10 | 0.2 | Konsistenzgeber |
| | Wasser entmin. u. konserv. | 10.0 | |
| | Pfirsischkernöl | 2.0 | |
| | Mandelöl | 3.0 | |
| | Olivenöl | 2.0 | |
| | Sojaöl | 1.5 | |
| | Lecithin | 1.5 | |
| | Cetylalkohol | 2.0 | 12 Gew.-% einer Lipidmischung aus pflanzlichen Ölen, Phospholipide, Fettalkohole |
| | α-Tocopherol mit Weinsäure | 0.8 | Fettstabilisator |
| F. | Molekulardestilliertes Glycerinmonostearat selbstemulgierend | 2.0 | Emulgator |
| G. | Lavendelöl | 0.5 | Wirkst./Parfümierung zur pH-Einstellung |
| H. | Milchsäure | | |

Die Herstellung der Zubereitung erfolgte in folgender Weise:

Die Bestandteile A wurden auf 70°C erwärmt und miteinander verrührt. Die Bestandteile B, C, D und E wurden jeweils separat vermischt und unter Rühren und weiterem Erwärmen nach und nach dem Gemisch der Bestandteile A zugesetzt. Die Bestandteile F wurden auf etwa 70°C erwärmt und danach langsam unter Rühren mit dem etwa 70°C warmen Gemisch der Bestandteile A, B, C, D und E versetzt. Das Rühren wurde fortgesetzt, bis das Gemisch auf etwa 40°C abgekühlt war. Anschließend wurde der Bestandteil G zugesetzt und der pH-Wert des so erhaltenen Gemisches mit dem Bestandteil H eingestellt.

### Beispiel 5

Aus den nachfolgend angegebenen Bestandteilen wurde eine Zubereitung hergestellt.

| **Bestandteile** | | **ca. Gew.-%** | **Funktion** |
|---|---|---|---|
| A. | Natriumlaurylethersulfat 70 %ig | 15.0 | 14.5 Gew.-% anionische waschaktive Substanzen |
| | Kondensationsprodukt von Eiweißhydrolysat aus natürlichem Kollagen mit Kokosfettsäuren 39 %ig | 10.0 | |
| B. | Natriumalginat | 0.5 | Konsistenzgeber |
| | Wasser entmin. u. konserv. | 10.0 | |
| C. | Wasser entmin. u. konserv. | 30.0 | |
| D. | Olivenöl | 4.5 | |
| | Erdnußöl | 4.5 | |
| | Sesamöl | 5.0 | |
| | Sojaöl | 6.0 | |
| | Kakaobutter | 2.0 | |
| | Unverseifbares der Avocado | 0.5 | 35 Gew.-% einer Lipidmischung aus pflanzlichen Ölen und Fetten, Phytosterine, Phospholipid, synthetischen tierischen Wachse, Fettalkohol |
| | Lecithin | 3.0 | |
| | Purcellinöl = (synthetisches Bürzeldrüsenöl) | 5.0 | |
| | Cetylalkohol | 3.5 | |
| | α-Tocopherol mit Weinsäure | 0.5 | Fettstabilisator |
| E. | Lavendelöl | 0.5 | Parfümierung |

Die Herstellung dieser Zubereitung wurde in folgender Weise durchgeführt:

Die Bestandteile A wurde auf 70°C erwärmt und miteinander vermischt. Die Bestandteile B wurden gemischt und unter Rühren dem Gemisch der auf 70°C erwärmten Bestandteile A zugesetzt. Anschließend wurde unter weiterem Erwärmen auf ca. 70°C und unter Rühren der Bestandteil C zugesetzt. Die Bestandteile D wurden auf 70°C erwärmt und anschließend langsam unter Rühren mit dem Gemisch der Bestandteile A, B und C versetzt. Die so erhaltene Emulsion wurde bis zur Abkühlung auf etwa 40°C gerührt und danach mit dem Bestandteil E versetzt.

### Beispiel 6

Aus den nachfolgenden Bestandteilen wurde eine weitere Zubereitung hergestellt.

| **Bestandteile** | | **ca. Gew.-%** | **Funktion** |
|---|---|---|---|
| A. | Saccharoseester mit Kokosfettsäureester 70 %ig | 75.0 | ca. 50 Gew.-% nichtionische waschaktive Substanz |
| B. | Olivenöl | 3.5 | 12.5 Gew.-% einer Lipidmischung aus pflanzlichen Ölen und Phospholipiden |
| | Sesamöl | 3.5 | |
| | Avocadoöl | 1.5 | |
| | Sojaöl | 2.0 | |
| | Lecithin | 2.0 | |
| | α-Tocopherol mit Weinsäure | 0.5 | |
| C. | Wasser entmin. u. konserv. | a.d. 100.0 | |

Die Herstellung der Zubereitung erfolgt in folgender Weise:

Die Bestandteile A und B wurden separat auf etwa 70°C erwärmt. Anschließend wurden die Bestandteile B langsam und unter Rühren mit dem Bestandteil A versetzt. Danach wurde der Bestandteil C zugesetzt und das so erhaltene Gemisch bis zur Abkühlung auf etwa 40°C gerührt.

### Beispiel 7

Aus den nachfolgenden Bestandteilen wurde eine weitere Zubereitung hergestellt.

| **Bestandteile** | | **ca. Gew.-%** | **Funktion** |
|---|---|---|---|
| A. | Kaliumseife auf der Basis von Leinöl, Fettsäure ca. 40 % | 30.0 | ca. 15 Gew.-% einer Kombination waschaktiver Substanzen |
| | Natriumlaurylethersulfat + 5 Gew.-% Betain 56 %ig | 10.0 | |
| B. | Wasser entmineralisiert | 33.0 | |
| | Konservierungsmittel | 0.3 | |
| | Methylhydroxycellulose | 0.3 | Ö/W Stabilisator |
| C. | Bienenwachs | 1.0 | 23.5 Gew.-% einer Lipidkombination aus pflanzlichen Ölen, tierischen und pflanzlichen Wachsen, Phospholipide, Fettalkohol |
| | Jojobaöl | 3.0 | |
| | Süßmandelöl | 5.0 | |
| | Pfirsischkernöl | 5.0 | |
| | Olivenöl | 5.0 | |
| | Lecithin | 2.0 | |
| | Cetylalkohol | 2.5 | |
| | α-Tocopherol mit Zitronensäure | 0.3 | Fettstabilisator |
| | Molekulardestilliertes Glycerinmonostearatat selbstemulgierend | 2.0 | Ö/W Emulgator |
| D. | Ätherische Öle: Lavendel, Zitrone, Eukalyptus, Salbei, Minze | 0.2 | Wirk./Parfümierung |
| E. | Zitronensäure | | zur pH-Einstellung |

Die Herstellung dieser Zubereitung erfolgte in folgender Weise:

Die Bestandteile A wurden auf 70°C erwärmt und miteinander verrührt. Die Bestandteile B wurden miteinander vermischt und unter Rühren dem Gemisch der Bestandteile A zugegeben. Die Bestandteile C wurden auf 70°C erwärmt und anschließend mit dem etwa 70°C warmen Gemisch der Bestandteile A und B langsam unter Rühren versetzt. Anschließend wurden die Bestandteile D zugesetzt und danach der pH-Wert der Emulsion mit dem Bestandteil E eingestellt.

### Beispiel 8

Zur Ermittlung der hautirritierenden Wirkung der Zubereitungen wurde die Zubereitung gemäß Beispiel 1 einem Epikutantest unterworfen. Dieser Test dient zum Nachweis einer primären Hautirritation bzw. einer Kontaktallergie, örtlich und zeitlich begrenzt, mit der zu untersuchenden Substanz.

Ein Kollektiv von 29 weiblichen und 21 männlichen Probanden (Alter: 18 bis 74 Jahre), darunter 10 Allergiker und 5 hautempfindliche Personen, nahmen an der Studie teil Als Testfeld diente der Rücken. Unter Verwendung eines handelsüblichen Testpflasters wurde eine 2 %ige wäßrige Lösung der Zubereitung auf die Haut aufgelegt und fixiert (Auftragsmenge: 2-5 mg der Zubereitung pro cm² Haut). Nach 48 Stunden wurde das Testpflaster entfernt und das Testfeld beurteilt. Weitere Beurteilungen erfolgten nach 72 Stunden. Die Beurteilung erfolgte gemäß folgendem Bewertungsschlüssel:

| | |
|---|---|
| 0 | keine Irritation |
| ± | schwaches oder zweifelhaftes Erythem |
| + | deutliches Erythem (auch urtikariell) |
| + + | starkes Erythem und/oder Papelbildung |
| + + + | dichtstehende Papeln und/oder Vesikeln |
| + + + + | Blasenbildung oder Nekrose |

Die bei der Untersuchung erhaltenen Ergebnisse sind in der Tabelle 1 zusammengestellt.

**Tabelle 1**

| Lfd. Nr. | Testperson | Alter | Geschl. | Diagnose | Reaktion | |
|---|---|---|---|---|---|---|
| | | | | | 48h | 72h |
| 1 | W.M. | 47 | w | hautempf. | 0 | 0 |
| 2 | B.L. | 52 | w | gesund | 0 | 0 |
| 3 | H.L. | 27 | w | gesund | 0 | 0 |
| 4 | H.T. | 27 | m | gesund | 0 | 0 |
| 5 | S.V. | 22 | m | gesund | 0 | 0 |
| 6 | S.E. | 21 | w | Atopiker | 0 | 0 |
| 7 | R.S. | 30 | w | hautempf. | 0 | 0 |
| 8 | R.J. | 35 | m | gesund | 0 | 0 |
| 9 | E.E. | 74 | w | gesund | 0 | 0 |
| 10 | P.R. | 39 | w | Atopiker | 0 | 0 |
| 11 | P.I. | 62 | w | hautempf. | 0 | 0 |
| 12 | S.R | 46 | w | gesund | 0 | 0 |
| 13 | S.L. | 43 | m | gesund | 0 | 0 |
| 14 | S.T. | 24 | m | gesund | 0 | 0 |
| 15 | G.M. | 24 | w | gesund | 0 | 0 |
| 16 | F.R. | 23 | w | gesund | 0 | 0 |
| 17 | F.T. | 24 | m | gesund | 0 | 0 |
| 18 | S.A. | 21 | w | gesund | 0 | 0 |
| 19 | K.M. | 23 | m | gesund | 0 | 0 |
| 20 | S.J. | 62 | m | gesund | 0 | 0 |
| 21 | S.R. | 68 | w | gesund | 0 | 0 |
| 22 | C.T. | 34 | m | gesund | 0 | 0 |
| 23 | C.S. | 35 | w | gesund | 0 | 0 |
| 24 | R.E. | 39 | w | Atopiker | 0 | 0 |
| 25 | R.U. | 40 | m | gesund | 0 | 0 |
| 26 | KK. | 71 | w | gesund | 0 | 0 |
| 27 | H.M | 41 | w | Atopiker | 0 | 0 |
| 28 | S.P | 33 | m | Atopiker | 0 | 0 |
| 29 | S.A. | 67 | w | gesund | 0 | 0 |
| 30 | G.M. | 41 | w | Atopiker | 0 | 0 |
| 31 | T.G. | 41 | w | Atopiker | 0 | 0 |
| 32 | T.M. | 42 | m | Atopiker | 0 | 0 |
| 33 | D.C. | 36 | w | gesund | 0 | 0 |
| 34 | D.U. | 33 | m | gesund | 0 | 0 |
| 35 | W.W. | 55 | m | hautempf. | 0 | 0 |
| 36 | W.R. | 53 | w | Atopiker | 0 | 0 |
| 37 | W.K. | 29 | w | hautempf. | 0 | 0 |
| 38 | W.J. | 27 | m | Atopiker | 0 | 0 |
| 39 | H.K. | 28 | m | gesund | 0 | 0 |
| 40 | S.G. | 62 | m | gesund | 0 | 0 |
| 41 | M.A. | 44 | w | gesund | 0 | 0 |
| 42 | P.R. | 63 | w | gesund | 0 | 0 |
| 43 | S.E. | 47 | w | gesund | 0 | 0 |
| 44 | S.H. | 43 | m | gesund | 0 | 0 |
| 45 | S.T. | 18 | m | gesund | 0 | 0 |
| 46 | S.B. | 40 | w | gesund | 0 | 0 |
| 47 | S.D. | 48 | m | gesund | 0 | 0 |
| 48 | S.S. | 18 | m | gesund | 0 | 0 |
| 49 | F.R | 55 | w | gesund | 0 | 0 |
| 50 | F.G. | 72 | w | gesund | 0 | 0 |

Wie aus den Ergebnissen ersichtlich, fanden sich weder nach 48 Stunden noch nach 72 Stunden positive oder zweifelhafte Reaktionen, so daß sich bei der Prüfung kein Hinweis für eine primär irritierende Wirkung der Zubereitung ergab. Auch ließ sich bei den Tests keine bereits bestehende Sensibilisierung durch die Zubereitung auslösen.

### Beispiel 9

Zur Ermittlung der hautirritierenden Wirkung der Zubereitung gemäß Beispiel 2 wurde der im Beispiel 8 beschriebene Epikutantest unter Verwendung der Zubereitung gemäß Beispiel 2 anstelle der Zubereitung gemäß Beispiel 1 wiederholt.

Bei diesem Test wurden für die Zubereitung gemäß Beispiel 2 die gleichen Ergebnisse erhalten, wie sie im Beispiel 8 für die Zubereitung gemäß Beispiel 1 beschrieben wurden.

### Beispiel 10

Untersuchung der fettregulierenden Wirkung der Zubereitungen.

Der Wirksamkeitsnachweis der fettregulierenden Wirkung der Zubereitung wurde folgendermaßen ermittelt:

Die fettregulierende Wirkung gemäß Beispiel 1 wurde mit Hilfe von zwei unabhängigen Meßmethoden, der Sebumeter-Methode und der Sebufix-Methode, bestimmt.

Die Prüfung erfolgte an 10 freiwilligen Probanden mit fettiger, zum Teil auch zu Akne neigender Haut. Über einen Zeitraum von 6 Wochen behandelten die Probanden einmal täglich die Gesichtshaut mit der Zubereitung gemäß Beispiel 1 und wandten zusätzlich, je nach Hautzustand ein- bis dreimal wöchentlich die Zubereitung gemäß Beispiel 2 an. Zur Dokumentation der fettregulierenden Wirkung wurden Sebumeter-Messungen vor Beginn der Untersuchung sowie nach deren Abschluß nach 6 Wochen durchgeführt. Ergänzend wurde hierzu anhand des Sebufix-Testes der Anteil an Fettflecken auf einer Spezialfolie bestimmt. Als Referenz einer unbehandelten Kontrolle dienten Messungen in der Halsregion, in der während der gleichen Zeit keine kosmetischen Behandlungen durchgeführt wurden.

Zur Bestimmung des Hautoberflächenfettes wurde die Sebumeter-Methode^{®} angewandt. Dieser Methode liegt das Prinzip des Fettfleckfotometers zugrunde, d.h. es wird die Lichtdurchlässigkeit einer mit Sebum (Fett) benetzten Folie gemessen. Zu diesem Zweck wird zuerst der Meßkopf, bestehend aus einer Kassette mit Spezialkunststoffolie, auf die zu messende Hautstelle gedrückt. Der Meßkopf enthält ein etwa 0,1 mm starkes mattiertes Kunststoffband zur Hautfettabnahme, welches durch ¼ Umdrehung den jeweiligen neuen Meßabschnitt (64 mm²) für die nächste Messung freigibt. Der benutzte Teil der Folie wird dabei in der Kassette aufgerollt. Unter dem Meßabschnitt der Folie befindet sich ein Spiegel, der mit der Folie ca. 1 mm aus dem Meßkopf herausragt. Dieser Spiegel ist über eine ca. 10 N starke Feder mit dem Gehäuse verbunden, so daß bei der Messung die Folie durch den Spiegel mit dieser Kraft auf die Haut gepreßt wird. Dadurch ist gewährleistet, daß immer ein gleichmäßiger Druck von 10 N an der Abnahmestelle der Haut herrscht.
Die Meßzahl (Andrückzeit) an der Haut beträgt 30 Sekunden und wird über eine eingebaute Uhr kontrolliert. Zur Auswertung des Fettgehaltes wird die Kassette anschließend in das Gehäuse geschoben und die Transparenz der Folie mittels einer Meßzelle gemessen. Das Ergebnis wird von einem Mikroprozessor ausgewertet und digital zur Anzeige gebracht. Je mehr Sebum vorhanden ist, d.h. also, je größer die Transparenz der Folie ist, desto größer ist der Wert auf der Anzeige. Der ermittelte Meßwert des Oberflächenfettes wird in µm pro cm² angegeben. Das Gerät besitzt eine serielle Standard-Schnittstelle und ist an einen PC angeschlossen. Die Meßdaten werden nach einer speziellen Software verarbeitet und mit einem eigenen Grafik- und Statistik-Programm ausgewertet.

Das Prinzip der Sebufix F16-Methode basiert auf einer milchigen Spezialfolie (4 cm²), bei der sich das Hautfett, wenn es mit der Folie in Berührung kommt, in die Mikroporen der Folie setzt und als "black spots" sichtbar wird. Zur Durchführung der Messung wird das Sebufix F16 mit der klebenden Seite auf einen Sondenkopf aufgebracht und die Sonde mit der Folie für eine Minute auf die zu messende Hautstelle aufgedrückt. Eine Feder im Sondenkopf sorgt für einen gleichmäßigen Druck (3 N) während der Messung. Nach Ablauf der Meßzeit wird die Sebufix-Folie vom Sondenkopf abgenommen und auf einen Spezialträgerrahmen aufgeklebt. Um Okklusionseffekte der Haut zu vermeiden, darf die Meßzahl nicht länger als eine Minute betragen. Zur Auswertung wird der Trägerrahmen mit der Folie in das Magazin eines Spezialprojektors eingelegt, in dem eine Lichtquelle und eine Schwarz/Weiß-CCD-Kamera mit Optik eingebaut ist, wo es bildanalytisch ausgewertet und auf dem Bildschirm als Negativ angezeigt wird, d.h. die mit Fett durchsetzten Poren der Folie sind als helle Flecken sichtbar, während die Grundfläche dunkelgrau ist. Bei der Auswertung wird die Grundfläche von der Gesamtfläche abgezogen, so daß die "black spots" als weiße Flecken übrigbleiben, die in Anzahl und Größe nach Pixel gemessen werden und danach in neun Kategorien nach Flächengröße aufgeteilt werde. Ein zugehöriges Säulendiagramm erscheint unterhalb des Bildes. Die absolute Zahl der Fettflecken, die Flächengröße der Kategorie in mm² und der prozentuale Anteil der Fettflecken an der Gesamtfläche werden unter dem Säulendiagramm angezeigt.

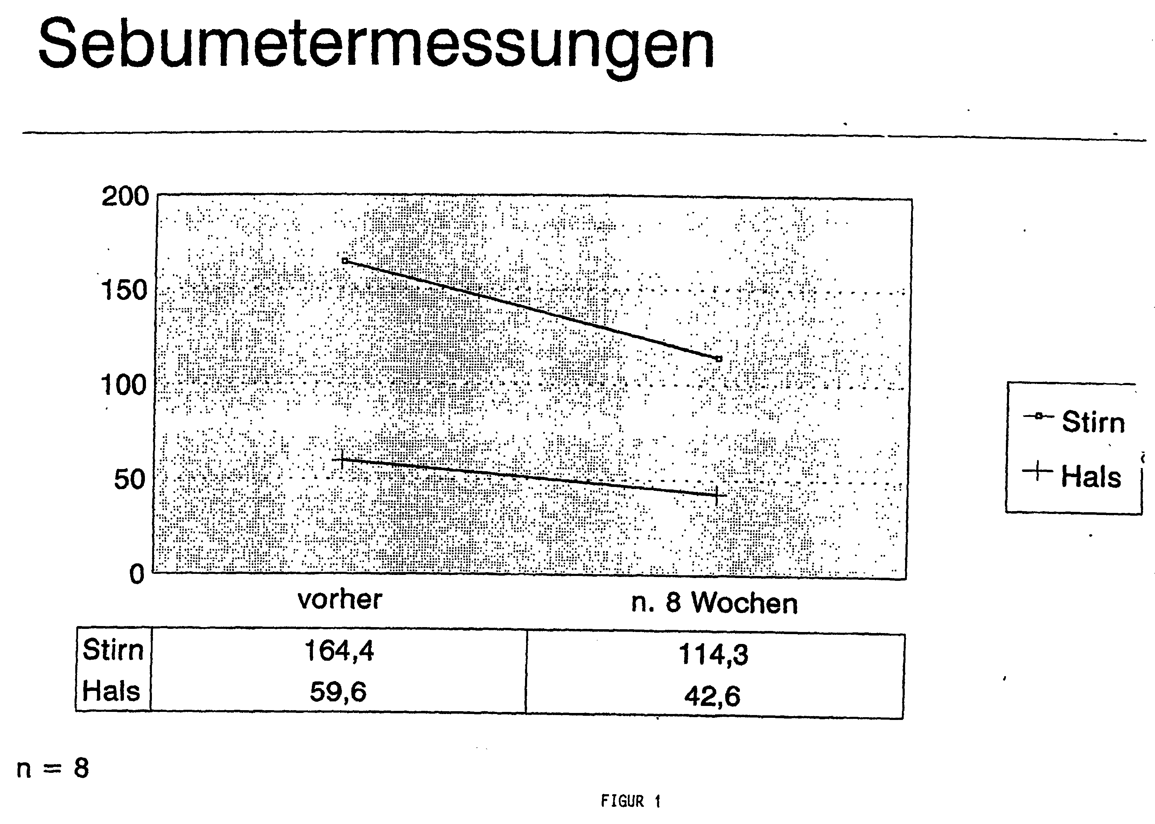

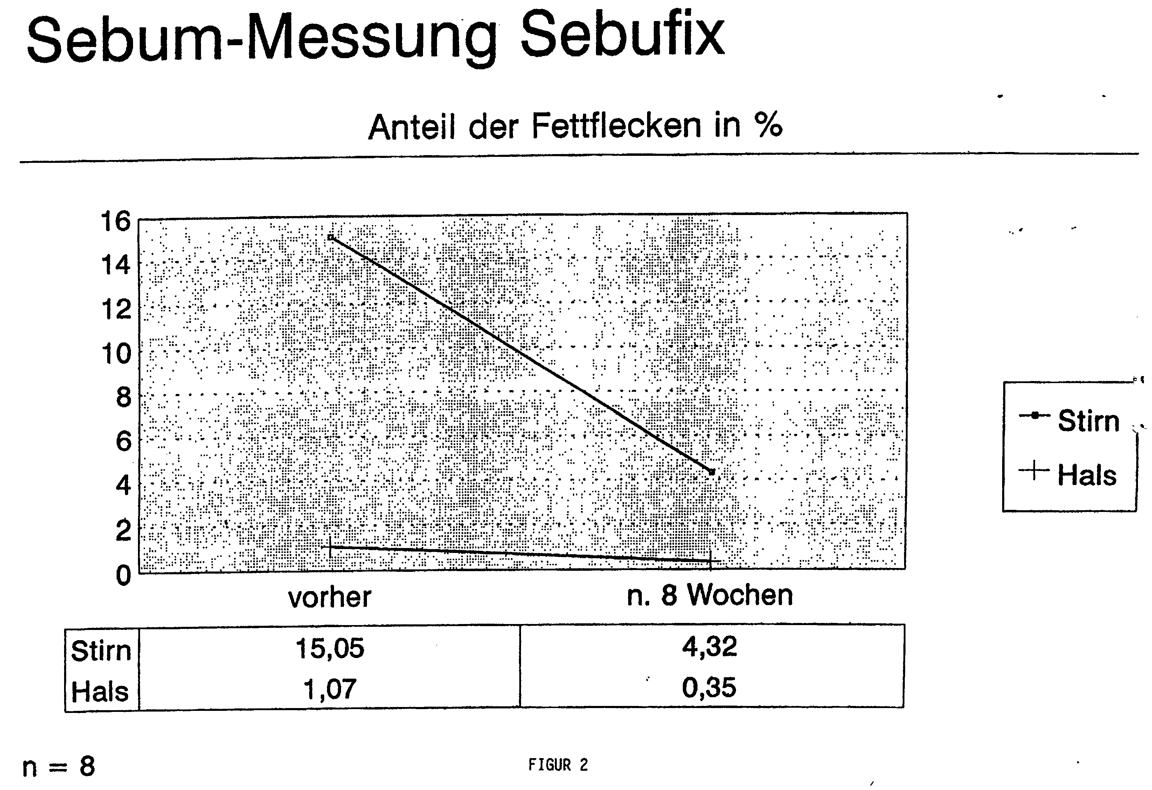

Die bei den beiden Untersuchungsmethoden erhaltenen Ergebnisse für die 10 an der Untersuchung teilnehmenden Personen sind in den Tabellen II und III zusammengefaßt und in den Figuren 1 und 2 grafisch dargestellt. Aus den Tabellen II und III sind die Meßdaten der einzelnen Personen zum Ausgangspunkt vor Erstapplikation nach 8 Wochen sowie die Mittelwerte aus den Ergebnissen für alle untersuchten Personen enthalten. Als Referenz einer unbehandelten Region diente der Halsbereich, der im gleichen Zeitraum keinerlei kosmetische Behandlungen erfuhr.

**Tabelle II**

| Anteil der Fettflecken in % | | | | |
|---|---|---|---|---|
| Probanden | Stirn vorher | nach 8 Wo | Hals vorher | nach 8 Wo |
| 1 | 5,21 | 0,47 | 1,2 | 1,84 |
| 2 | 16,89 | 6,59 | 3,76 | 0,1 |
| 3 | 4,18 | 0,89 | 0,68 | 0,49 |
| 4 | 3,75 | 7,14 | 0,12 | 0,14 |
| 5 | 13,67 | 7,37 | 0,02 | 0,01 |
| 6 | 8,62 | Abbruch | 0,02 | Abbruch |
| 7 | 4,53 | Abbruch | 0,02 | Abbruch |
| 8 | 4,77 | 0,1 | 0,85 | 0,05 |
| 9 | 62,62 | 11,89 | 1,89 | 0,12 |
| 10 | 9,28 | 0,11 | 0,02 | 0,01 |
| | | | | |
| Mittelwert (n = 8) | 15,05 | 4,32 | 1,07 | 0,35 |

**Tabelle III**

| Sebumetermessungen | | | | |
|---|---|---|---|---|
| Probanden | Stirn vorher | nach 8 Wo | Hals vorher | nach 8 Wo |
| 1 | 152 | 97 | 93 | 108 |
| 2 | 172 | 171 | 79 | 22 |
| 3 | 177 | 53 | 41 | 33 |
| 4 | 154 | 161 | 81 | 59 |
| 5 | 181 | 119 | 63 | 50 |
| 6 | 154 | Abbruch | 51 | Abbruch |
| 7 | 161 | Abbruch | 50 | Abbruch |
| 8 | 153 | 75 | 65 | 29 |
| 9 | 176 | 176 | 23 | 18 |
| 10 | 150 | 62 | 32 | 22 |
| | | | | |
| Mittelwert (n = 8) | 164,4 | 114,3 | 59,6 | 42,6 |

Wie aus den Untersuchungsergebnissen ersichtlich ist, konnte mit Hilfe von beiden Methoden eine fettregulierende Wirkung durch die Behandlung mit den Zubereitungen nachgewiesen werden. Im Falle der Sebumeter-Messungen sanken die Werte im Mittel von 164.4 auf 114.3 µg pro cm². Das entspricht einer Verminderung der Fettproduktion um etwa 30 %.

Die gleiche Tendenz zeigte sich auch bei der Untersuchung gemäß der Sebufix-Methode. Hier konnte der prozentuale Anteil der von Fett benetzten Fläche der Spezialfolie von 15,05 % auf 4,32 % gesenkt werden. Die Befunde entsprachen auch dem subjektiven Empfinden der Probanden, die eine Verminderung des Hautfettes, speziell in der T-Zone angaben. Es konnte festgestellt werden, daß bei den zur Auswertung herangezogenen Probanden eine deutlich fettregulierende Wirkung durch die Anwendung der Zubereitung auftrat.

## Patentansprüche

1. Verwendung einer gleichzeitig talglösend und pflegend wirkenden Zubereitung, die neben mindestens 25 Gew.-% Wasser und mindestens 5 Gew-% mindestens einer anionischen, amphoteren oder nicht-ionischen waschaktiven Substanz, 5 bis 60 Gew.% mindestens eines Lipids, ausgewählt aus der Gruppe bestehend aus pflanzlichen und/oder tierischen Öle, Fette und Wachse oder deren synthetischen Ersatzprodukte oder Derivate, Phospholipide oder deren synthetische Ersatzprodukte, Fettalkohole und Sterole, enthält und als abspülbare Öl-in-Wasser-Emulsion oder als amphiphile Emulsion vorliegt, zur Entfernung und Vermeidung von Comedonen und dadurch zur Akneprophylaxe.

2. Verwendung nach Anspruch 1, dadurch gekennzeichnet, daß die Zubereitung als Lipid pflanzliche und/oder tierische Öle und Fette oder deren synthetische Ersatzprodukte oder Derivate, vorzugsweise Ölivenöl, Avocadoöl, Mandelöl, Weizenkeimöl, Sheabutter, Nerzöl oder Walratöl enthält.

3. Verwendung nach Anspruch 1, dadurch gekennzeichnet, daß die Zubereitung als Lipid pflanzliche oder tierische Wachse und deren synthetische Ersatzprodukte oder deren Derivate, vorzugsweise Jojobaöl, Bienenwachs, Purcellinöl oder Walratersatz, enthält.

4. Verwendung nach Anspruch 1, dadurch gekennzeichnet, daß die Zubereitung als Lipid Fettalkohole oder Sterole, vorzugsweise Cetylalkohol oder Phytosterine, enthält.

5. Verwendung nach Anspruch 1, dadurch gekennzeichnet, daß die Zubereitung als Lipid Phospholipoide oder deren synthetische Ersatzprodukte, vorzugsweise Lecithine, enthält.

6. Verwendung nach einem oder mehreren der Ansprüche 1 bis 5, dadurch gekennzeichnet, daß die Zubereitung als Lipid ein Gemisch aus zwei oder mehreren der Lipide gemäß einem oder mehreren der Ansprüche 2 bis 5 enthält.

7. Verwendung nach einem der Ansprüche 1 bis 6, dadurch gekennzeichnet, daß die Zubereitung 25 bis 75 Gew.-% Wasser enthält.

8. Verwendung nach einem der Ansprüche 1 bis 7, dadurch gekennzeichnet, daß die Zubereitung einen Emulgator oder mehrere Emulgatoren, vorzugsweise Monofettsäureester mehrwertiger Alkohole oder Glycerinmono/di-stearat enthält.

9. Verwendung nach einem der Ansprüche 1 bis 8, dadurch gekennzeichnet, daß die Zubereitung zur Verdickung und Stabilisierung der Wasserphase Hilfs- und Zusatzstoffe, vorzugsweise Methylcellulose, Alginate oder Carragenate, enthält.

10. Verwendung nach einem der Ansprüche 1 bis 9, dadurch gekennzeichnet, daß die Zubereitung keratolytische, antibakterielle und/oder die Haut weichmachende Wirkstoffe enthält.

11. Verwendung nach einem der Ansprüche 1 bis 10, dadurch gekennzeichnet, daß die Zubereitung natürliche und/oder synthetische Schleifmittel vorzugsweise Polyethylengranulat oder Nußschalengranulate, enthält.

12. Verwendung nach einem der Ansprüche 1 bis 11, dadurch gekennzeichnet, daß die Zubereitung Mittel zur pH-Einstellung, vorzugsweise α-Hydroxysäuren, wie Milchsäure, Fruchtsäuren oder lecithinähnliche Phosphorsäureester, enthält.

13. Verwendung nach einem der Ansprüche 1 bis 12, dadurch gekennzeichnet, daß die Zubereitung ätherische Öle mit fungizider und/oder bakterizider Wirkung enthält.

## Claims

1. Use of a preparation both for removal of sebum and for protective skin care, comprising in addition to min. 25% by weight water and min. 5% by weight anionic, amphoteric or non-ionic active washing substance, 5 to 60% by weight min. of a lipid, selected from the group consisting of vegetable and/or animal oils, fats and waxes or synthetic substitutes or derivatives thereof, phospholipids and synthetic substitutes thereof, fatty alcohols and sterols, and is available as a rinseable oil-in-water emulsion or as an amphiphilic emulsion for the removal and prevention of comedones and thus as acne prophylaxis.

2. Use according to Claim 1, wherein the preparation as a lipid contains vegetable and/or animal oils and fats or synthetic substitutes or derivatives thereof, preferably olive oil, avocado oil, almond oil, wheat germ oil, shea butter, mink oil or sperm oil.

3. Use according to Claim 1, wherein the preparation as a lipid contains vegetable or animal waxes and synthetic substitutes or derivatives thereof, preferably jojoba oil, beeswax, purcellin oil or sperm oil substitute.

4. Use according to Claim 1, wherein the preparation as a lipid contains fatty alcohols or sterols, preferably cetyl alcohol or phytosterines.

5. Use according to Claim 1, wherein the preparation as a lipid contains phospholipids or synthetic substitutes thereof, preferably lecithin.

6. Use according to one or more of Claims 1 to 5, wherein the preparation as a lipid contains a compound of two or more lipids according to one or more of the Claims in 2 to 5.

7. Use according to one of the Claims in 1 to 6, wherein the preparation contains 25 to 75% by weight water.

8. Use according to one of the Claims in 1 to 7, wherein the preparation contains one emulsifier or several emulsifiers, preferably mono fatty acid esters, polyvalent alcohols or glycerine mono/distearate.

9. Use according to one of the Claims in 1 to 8, wherein the preparation contains auxiliary or supplementary substances, preferably methyl cellulose, alginate or carragenate to thicken and stabilise the aqueous phase.

10. Use according to one of the Claims in 1 to 9, wherein the preparation contains keratolytic, antibacterial and/or active ingredients which soften the skin.

11. Use according to one of the Claims in 1 to 10, wherein the preparation contains natural and/or synthetic abrasives, preferably polyethylene granulate or nutshell granulate.

12. Use according to one of the Claims in 1 to 10, wherein the preparation contains substances for pH regulation, preferably alpha hydroxy acids, such as lactic acid, fruit acids or phosphoric acid esters similar to lecithin.

13. Use according to one of the Claims in 1 to 10, wherein the preparation contains essential oils with fungicidal and/or bactericidal effect.

## Revendications

1. Utilisation d'une préparation qui dissout le suif et exerce en même temps une action soignante et dont les composants sont au moins 25 % en poids d'eau et au moins 5 % en poids d'une substance anionique, amphotère ou non ionique savonneuse, au moins entre 5 et 60 % d'une lipide sélectionnée dans le groupe comprenant des huiles soit végétales ou animales, des graisses et des cires ou leurs succédanés synthétiques ou leurs dérivés, des lipides phosphoriques ou leurs succédanés synthétiques, des alcools gras et des stérols. Cette préparation se présente sous forme soit d'une émulsion huile dans l'eau rinçable ou d'une émulsion amphiphile et a pour objet l'élimination et la prévention des comédons, exerçant ainsi une action prophylactique sur l'acné.

2. Utilisation selon la revendication 1, **caractérisée par le fait** que la préparation, en tant que lipide, contient des huiles et des graisses végétales et/ou animales ou leurs succédanés synthétiques ou leurs dérivés, de préférence l'huile d'olive, l'huile d'avocat, l'huile d'amande, l'huile de germes de blé, le beurre de karité, l'huile de vison ou l'huile de blanc de baleine.

3. Utilisation selon la revendication 1, **caractérisée par le fait** que la préparation, en tant que lipide, contient des cires soit végétales ou animales ou leurs succédanés synthétiques ou leurs dérivés, de préférence l'huile jojoba, la cire d'abeille, l'huile purcellin ou un succédané de blanc de baleine.

4. Utilisation selon la revendication 1, **caractérisée** par le fait que la préparation, en tant que lipide, contient soit des alcools gras ou des stérols, de préférence de l'alcool cétylique ou du phylostérol.

5. Utilisation selon la revendication 1, **caractérisée par le fait** que la préparation, en tant que lipide, contient des lipoïdes au phosphore ou leurs succédanés synthétiques, de préférence de la lécithine.

6. Utilisation selon l'une ou plusieurs des revendications 1 à 5, **caractérisée par le fait** que la préparation, en tant que lipide, contient un mélange de deux ou de plusieurs des lipides suivant une ou plusieurs des revendications 2 à 5.

7. Utilisation selon l'une ou plusieurs des revendications 1 à 6, **caractérisée par le fait** que la. préparation contient entre 25 et 75 % en poids d'eau.

8. Utilisation selon l'une ou plusieurs des revendications 1 à 7, **caractérisée par le fait** que la préparation contient un ou plusieurs émulsionnants, de préférence un ester monoacide gras d'alcools polyvalents ou un mono/di-stéarate glycéride.

9. Utilisation selon l'une ou plusieurs des revendications 1 à 8, **caractérisée par le fait** que la préparation contient des auxiliaires et des additifs, de préférence de la cellulose méthylique, de l'alginate ou du carraghénate, pour l'épaississement et la stabilisation de la phase eau.

10. Utilisation selon l'une ou plusieurs des revendications 1 à 9, **caractérisée par le fait** que la préparation contient des substances actives kératolytiques et antibactérielles et/ou des adoucissants pour la peau.

11. Utilisation selon l'une ou plusieurs des revendications 1 à 10, **caractérisée par le fait** que la préparation contient des abrasifs naturels et/ou synthétiques, de préférence des granulés polyéthylènes ou des granulés de coquilles de noix.

12. Utilisation selon l'une ou plusieurs des revendications 1 à 11, **caractérisée par le fait** que la préparation contient des produits de réglage de pH, de préférence des acides hydroxyliques a, tels que les acides lactiques, les acides de fruits ou les esters phosphoriques analogues à la lécithine.

13. Utilisation selon l'une ou plusieurs des revendications 1 à 12, **caractérisée par le fait** que la préparation contient des huiles essentielles à effet fongicide et/ou bactéricide.
